# EUROPEAN PATENT SPECIFICATION

(11) **EP 3 166 510 B1**
(45) Date of publication and mention of the grant of the patent: **16.05.2018**
(21) Application number: 15729907.4
(22) Date of filing: 10.06.2015
(51) Int. Cl.: A61B 17/15, A61B 17/16, A61B 17/17, A61F 2/46

(54) **APPARATUS FOR SURGICALLY REPLACING A HUMAN ANKLE JOINT**
VORRICHTUNG ZUM CHIRURGISCHEN ERSETZEN EINES MENSCHLICHEN SPRUNGGELENKS
APPAREIL POUR LE REMPLACEMENT CHIRURGICAL D'UNE ARTICULATION TIBIO-TARSIENNE HUMAINE

(30) Priority: 10.07.2014 GB 201412261
(43) Date of publication of application: 17.05.2017
(73) Proprietor: Ortho Solutions UK Limited, Maldon Chelmsford Essex CM9 6FF (GB)
(72) Inventor: STAMP, Kevin, Chelmsford Essex CM9 6FF (GB)
(74) Representative: Stratagem IPM Limited
(86) International application number: PCT/GB2015/051711
(87) International publication number: WO 2016/005722

(56) References cited:
- US-A1- 2004 039 394
- US-A1- 2006 142 870
- US-A1- 2008 306 605
- US-A1- 2012 130 376

## Description

### FIELD

The present invention relates to apparatus for surgically replacing a human ankle joint.

### BACKGROUND

The human ankle joint is highly complex and is required to be strong enough to bear the entire weight of the human body while retaining sufficient flexibility and motion to permit the ankle to be rotated and pivoted. Due to circumstances such as injury, disease and wear, for example, a person's ankle can cause debilitating pain and restriction of movement. In such circumstances it might become necessary to replace a patient's ankle with an artificial ankle joint to restore mobility and, in many cases, relieve pain..

Replacement of a patient's ankle requires an invasive surgical procedure which involves replacing parts of the person's skeletal system. Conventional methods of surgery involve removing both sides of the ankle joint and replacing with a prostheses, or implant. The prostheses replaces the ankle socket which is part of the tibia and the top of the talus bone.

In one prior art surgical procedure a person's tibia and fibula are cut to create a channel bounded by the fibula and a portion of the tibia. The cuts made in the person's tibia, fibula and talus create an opening bounded on four sides by bone. The prostheses is inserted into the opening and held in place by a tigh fit between the person's bones.

As the prostheses is held in place partly by the tibia and fibula which can move, to some extent, independently of one another, bone graft is placed between the tibia and the fibula to create a fusion therebetween to prevent independent movement of the two bones. A bone plate is also secured to the fibula by two screws which pass through the fibula and the tibia.

Such a procedure often necessitates further surgery to medially and laterally align the prosthesis.

US2012/0130376 and US2006/0142870 each describe further apparatus for replacing a human ankle joint.

The present invention seeks to provide improved apparatus for and an improved method of replacing a human ankle joint.

### SUMMARY

The invention is defined in claims 1 and 14. A first aspect of the invention provides an apparatus for replacing a human ankle joint, the apparatus comprising:
a positioning jig for setting a position of a tool holder relative to a part of a person's anatomy;
a cyclical saw removably mountable to the tool holder for making a distal tibial cut though the person's tibia bone;
a talus positioning guide removably mountable to the tool holder for setting a position of location holes to be drilled in the person's talus bone;
a talus re-section guide, alignable with the location holes to be drilled in the person's talus bone, for providing a cutting face for making a superior talar cut through the person's talus bone;
an ankle alignment jig for setting the position of one or more slots in the person's tibia bone, the said one or more slots being configured to receive a first portion of an artificial ankle joint, and for setting the position of one or more guide holes to be drilled in the person's talus bone in order to facilitate medial,lateral, posterior and anterior alignment of the first portion of the artificial ankle joint with a second portion of the artificial ankle joint;
a talus drill template comprising one or more dowels, or pins, configured to be received by the one or more guide holes to be drilled in the person's talus bone, the talus drill template further comprising one or more drill guides for setting the position of at least one further hole to be drilled in the person's talus bone;
a talus roof top preparation guide comprising at least two dowels, or pins, configured to be received respectively by the one or more guide holes to be drilled in the person's talus bone and by the at least one further hole to be drilled in the person's talus bone, the roof top preparation guide further comprising two large diameter holes for receiving a cutting tool for removing a portion of the person's talus bone;
a talus routing guide for setting the position of one or more slots in the person's talus bone, the said one or more slots being configured to receive the second portion of the artificial ankle joint; and
a manipulator for positioning the artificial ankle joint between the person's tibia and talus bones, the manipulator comprising a handle portion and a body portion, wherein the body portion comprises at least one mounting point configured to engage with the artificial ankle joint and a direction guide configured to prevent the artificial ankle joint from being incorrectly inserted into the manipulator.

The apparatus of the first aspect of the invention permits a surgeon to align an artificial ankle joint in the medial, lateral, anterior and posterior aspects. Due to the complexity of the human ankle joint, it is critical that the artificial ankle joint is aligned. Any error in alignment during implantation is likely to result in the need for further surgery to re-align the artificial ankle joint, as is common in the prior art.

The first aspect of the invention provides a tool positioning jig to ensure that the distal tibial and superior talar cuts made through the person's tibia and talus bones respectively are taken from a base, or zero, location. This ensures that a gap of the correct width, and in the correct position, is created between the person's tibia and talus bones.

The width of the gap created between the person's tibia and talus bones is critical to enable the ankle positioning jig to be inserted therein. The position of the gap between the person's tibia and talus bones is critical to set the position of structural modifications to be made to the person's talus bone. The ankle positioning jig is inserted into the gap between the person's tibia and talus bones and sets the position of guide holes to be drilled in the person's talus bone. The guide holes, when drilled in the person's talus bone, are used to set the position of all further modifications to the person's talus bone.

The tool positioning jig and the ankle positioning jig between them link all modifications to the person's tibia and talus bones to the base, or zero, location. All modifications are thus linked to the distal tibial and superior talar cuts and the position of the guide holes in a person's talus bone set by the ankle positioning jig. This has the effect of accurately positioning and aligning all structural modifications made to the person's tibia and talus bones to reliably attain medial, lateral, anterior and posterior alignment of the artificial ankle joint.

Posterior and anterior alignment is effected by controlling the depth of the slots to be cut into the person's tibia and talus bones. A routing tool is inserted through oval apertures in the ankle positioning jig to cut the slots in the person's tibia bone and through oval apertures in the talus routing guide to cut the slots in the person's talus bone. Both sets of slots are cut to a pre-determined depth. The ankle positioning jig and talus routing guide each provide a surface to limit the depth to which the routing tool can cut into the person's tibia and talus bones. Different depths of slot can be cut depending on how the routing tool is configured.

A second aspect of the invention provides a tool configured for positioning an artificial ankle joint between a human tibia and talus, the tool comprising: a handle portion and a body portion, wherein the body portion comprises a pair of diametrically opposed engagement features configured to engage with an artificial ankle joint, and a direction guide configured to prevent the artificial ankle joint from being incorrectly inserted into the tool.

Currently used methods and apparatus for replacing human ankle joints have no fail safe way of preventing a surgeon from installing an artificial ankle joint into a patient back to front. If an artificial ankle joint is installed incorrectly further traumatic surgery would be required to correct the error. The invention provides a direction guide which is configured to only receive the artificial ankle joint when orientated in a pre-determined direction. The engagement features may comprise substantially symmetrical protrusions or indents.

The use of protrusions or indents enables the artificial ankle joint to snap fit into the tool with minimal effort and time for the surgeon.

The tool may further comprise a release mechanism for releasing the artificial ankle joint from the engagement features.

The direction guide may comprise a third engagement feature on the body of the tool angularly separated from the pair of diametrically opposed engagement features of the body portion of the tool. The third engagement feature may be angularly separated from the diametrically opposed engagement features by approximately ninety degrees.

The tool may have a curvilinear profile configured to substantially correspond to the profile of an artificial ankle joint.

### BRIEF DESCRIPTION OF THE DRAWINGS

Certain embodiments will now be described with reference to the following drawings.
Figure 1 shows an illustrative view of an artificial ankle joint according to the present invention implanted between a person's tibia and talus;
Figure 2a shows an illustrative view of a talus facing surface of a tibia attachment portion of the artificial ankle joint according to figure 1;
Figure 2b shows an illustrative view of a tibia facing surface of the tibia attachment portion of the artificial ankle joint according to figures 1 and 2a;
Figure 3a shows an illustrative view of a tibia facing surface of a talus attachment portion of the artificial ankle joint according to figure 1;
Figure 3b shows an illustrative view of a talus facing surface of the talus attachment portion of the artificial ankle joint according to figures 1 and 3a;
Figure 4a shows an illustrative view of a tool positioning jig used to create parallel cuts in a person's tibia and talus;
Figure 4b shows a detailed view of a tool holder mounted at the distal end of the positioning jig of figure 4a;
Figure 5 shows an illustrative view of a talus positioning guide mountable to the tool holder of figure 4b and used to locate the talus re-section block of figure 6;
Figure 6 shows an illustrative view of a talus re-section guide mountable to the tool holder of figure 4b and used to define the location of the second cut through the person's talus;
Figure 7a shows top and front surfaces of an ankle positioning jig used to align the tibia attachment portion and talus attachment portion of the artificial ankle joint in the medial, lateral, anterior and posterior aspects;
Figure 7b shows bottom and rear surfaces of the ankle positioning jig of figure 7a;
Figure 8 shows an illustrative view of a talus drill template used to create holes in the person's talus for receiving a part of a talus roof top preparation guide as illustrated in figure 9;
Figure 9 shows an illustrative view of a talus roof top preparation guide used to define a portion of the patients talus to be removed prior to implantation of the artificial ankle joint of figures 1 to 3b;
Figure 10 shows an illustrative view of a talus routing guide used to define a location of two slots to be cut in the person's talus;
Figure 11 shows an illustrative view of a rotary cutting tool used to remove a portion of the person's talus;
Figure 12 shows an illustrative view of an artificial ankle joint manipulator used to receive the artificial ankle joint of figures 1 to 3b and aid a surgeon in locating the artificial joint between the person's tibia and talus;

### DETAILED DESCRIPTION OF THE CERTAIN EMBODIMENTS

The certain embodiments will now be described, by way of example only, with reference to the brief description of the drawings.

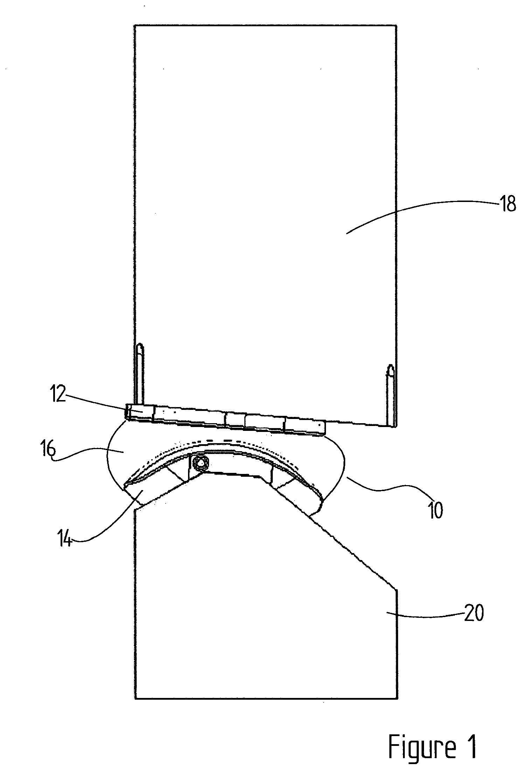

An artificial ankle joint 10 according to an embodiment of the present invention is illustrated in figures 1 to 3b. The artificial ankle joint 10 comprises a tibia attachment portion 12 and a talus attachment portion 14. A bearing 16 is located between the tibia attachment portion 12 and talus attachment portion 14 of the artificial ankle joint 10. The tibia attachment portion 12 engages with a person's tibia 18 and the talus attachment portion 14 engages with the person's talus 20, as will be described. The bearing 16 permits the person's talus 20 to move relative to the tibia 18 in three axes of movement within a pre-determined range of movement.

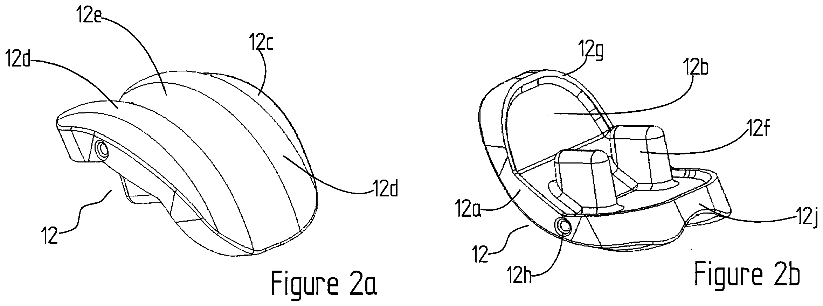

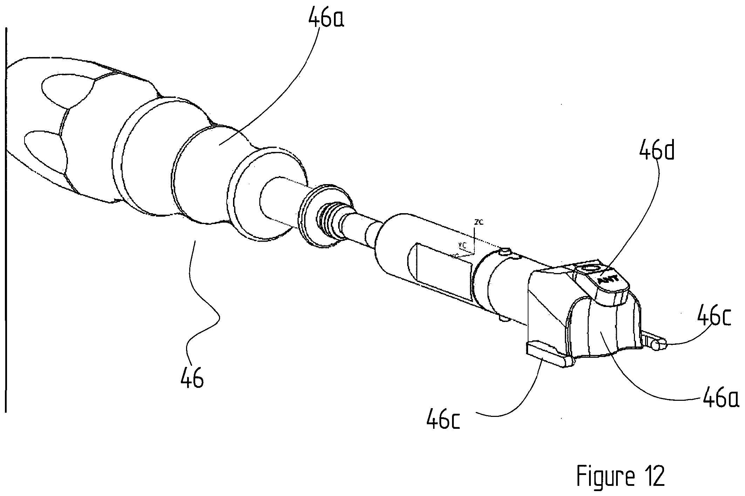

The talus attachment portion 14 is shown in figures 2a and 2b and comprises a body 12a having a talus facing surface 12b and a tibia facing surface 12c. The body 12a has a thickness between the talus facing surface 12b and tibia facing surface 12c. Two diametrically opposed mounting points 12h are provided on the body 12a for engagement with an artificial ankle joint manipulator 46, as will be described with reference to figure 12. In the illustrated embodiment the mounting points 12h comprise dents, or dimples, in the body 12a which would engage with corresponding protrusions 46c on the artificial ankle joint manipulator 46. Alternatively, it will be appreciated that the mounting points 12h could comprise protrusions from the body 12a which would engage with corresponding dents, or dimples, on the artificial ankle joint manipulator 46. It will also be appreciated that other means of mounting the artificial ankle joint 10 into the manipulator could be used and the aforegoing description does not limit the invention to the embodiment described.

The tibia facing surface 12c comprises two protuberances 12d defining a channel 12e therebetween. The protuberances 12d and channel 12e extend curvilinearly substantially entirely along the longitudinal length of the talus attachment portion 12. The channel 12e assists in holding the bearing 16 in place between the talus attachment portion 12 and tibia attachment portion 14 by constraining range of movement of the bearing 16 and thus the person's talus 20 relative to the person's tibia 18.

The talus facing surface 12b of the talus attachment portion 12 is provided with two talus engagement members 12f, or kiels, protruding substantially perpendicular from said talus facing surface 12b. Each talus engagement member 12f engages with a respective slot cut into the person's talus 20, as will be described. The talus facing surface 12b of the talus attachment portion 12 is further provided with a circumferential ridge, or lip, 12g which stands up from the talus facing surface 12b. A lateral cross-section of the person's talus 20 created by a surgeon fits within the circumferential ridge or lip 12g

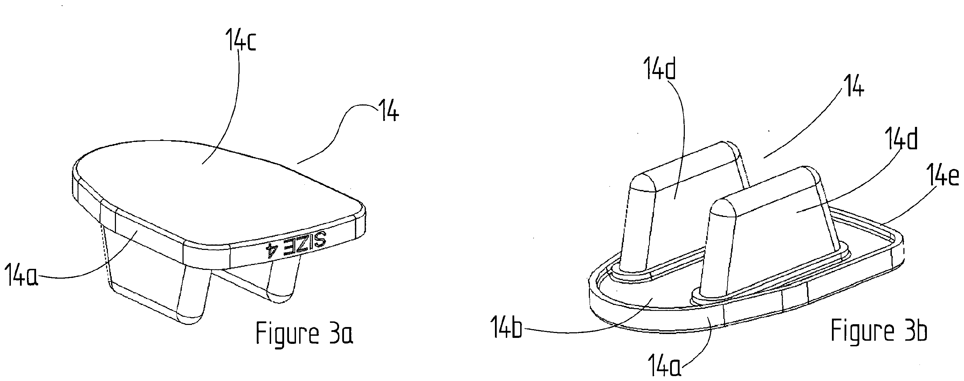

The tibia attachment portion 14 is shown in figures 3a and 3b and is configured to broadly correspond in shape to a lateral cross-section of the person's tibia 18. The tibia attachment portion 14 comprises a body 14a having a tibia facing surface 14b and a talus facing surface 14c. The talus facing surface 14c is substantially flat.

Two tibia engagement members, or kiels, 14d protrude substantially perpendicularly from the tibia facing surface 14b. The tibia engagement members 14d each engage with respective slots cut into the person's tibia 18, as will be described. The tibia facing surface 14b further comprises a circumferential ridge, or lip, 14e which stands up from the tibia facing surface 14b. The lateral cross-section of the person's tibia fits within the circumferential ridge, or lip 14e.

The talus facing surface 12b of the talus attachment portion 12 and the tibia facing surface 14b of the tibia attachment portion 14 have surface treatments of a specification configured to encourage bone growth thereon. It will be appreciated that many different surface treatments could be chosen for this purpose including but not limited to pitting, surface ridges and roughness, for example. The tibia facing surface 12c of the talus attachment portion 12 and the talus facing surface 14c of the tibia attachment portion 14 have surface treatments of a specification configured to deter bone growth thereon. Such a surface could include, but is not limited to, a highly polished surface.

The bearing 16 is positioned between the talus attachment portion 12 and the tibia attachment portion 14 of the artificial ankle joint 10. The bearing 16 allows the talus attachment portion 12 to move relative to the tibia attachment portion 14 between a specified range of motion in three dimensions. The bearing 16 is constrained only by the relative position of the talus attachment portion 12 and tibia attachment portion 14 of the artificial ankle joint 10. The bearing 16 can thus slide along the curvilinear channel 12e of the talus attachment portion 12 of the artificial ankle joint 10 and pivot within said channel 12e.

The artificial ankle joint 10 is implanted into a patient utilising a number of surgical tools each of which will now be described.

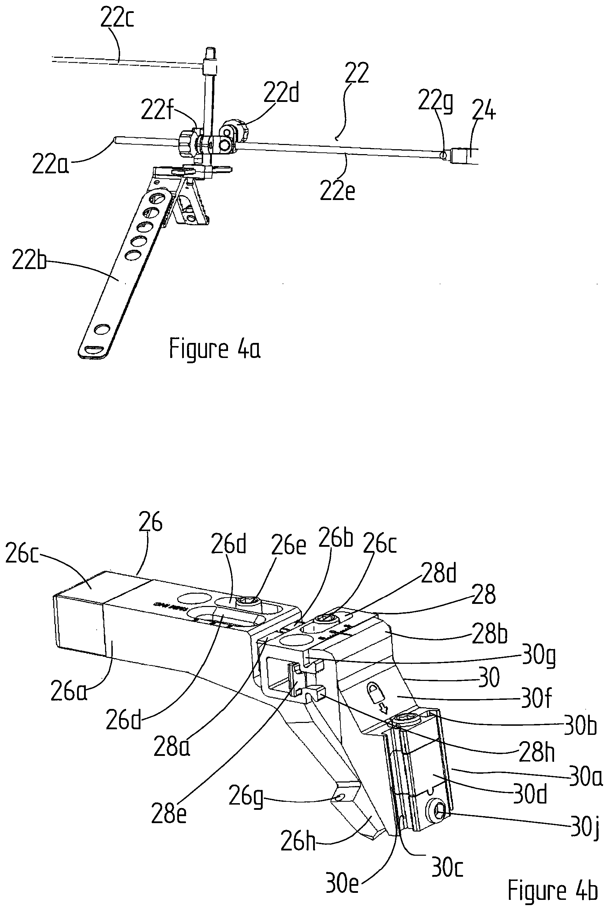

A tool positioning jig 22 is attached to the person's leg before surgery. The tool positioning jig 22 is used to ensure that accurate cuts are taken from and holes drilled into the person's tibia 18 and talus 20 to enable a surgeon to correctly implant and align the artificial ankle joint 10. As shown in figure 4a, a proximal end 22a of the tool positioning jig 22 is attached to a person's leg by either strapping the tool positioning jig 22 around the person's knee or by drilling one or more holes into the person's knee cap for insertion of one or more pins (not shown). The illustrated embodiment has a strap 22b which can be positioned around the person's knee and tightened to hold the proximal end 22a of the tool positioning jig 22 in the required position.

The tool positioning jig 22 is aligned with the person's tibia 18 by manual manipulation of a manipulating rod 22c and once strapped to the person's knee can be angularly orientated by the surgeon by way of one or more adjustment devices 22d which permit the positioning jig to rotate around the person's leg and/or be offset from alignment with the person's tibia 18. The longitudinal length of the tool positioning jig 22 can also be adjusted in accordance with the height of the patient and length of the person's tibia 18. Adjustment of the longitudinal length of the tool positioning jig 22 can be effected by movement of a rod 22e relative to the person's knee. The position of the rod 22e is fixed when an adjustment device 22f is locked and movable when the adjustment device 22f is unlocked. Alternatively, the longitudinal length of the positioning jig 22 could be adjusted by way of a telescopic pole, or rod (not shown).

The distal end 22g of the tool positioning jig 22 is provided with a tool holder 24, as will be described in detail with reference to figure 4b, which is mounted to the rod 22e. The tool holder 24 comprises three distinct portions: a longitudinal adjustment portion 26 a lateral adjustment portion 28 and a tool mount 30.

The longitudinal adjustment portion 26 of the tool holder 24 is fixedly mounted to the rod 22e and comprises a substantially hollow block 26a having an opening 26b to receive at least part of the lateral adjustment portion 28 of the tool holder 24. The hollow block 26a is separated into two chambers by a longitudinal divider (not shown) running rearwardly from the opening 26b into the hollow block 26a. The top surface 26c of the longitudinal adjustment portion 26 comprises two obround apertures 26d, one acting as a position indicator window and the other for receiving a mechanical fastener 26e to fix the longitudinal position of the lateral adjustment portion 28 relative to the longitudinal adjustment portion 26. A position guide (not shown) is located adjacent to the obround aperture 26d used as a position indicator window.

The longitudinal adjustment portion 26 further comprises an angled body portion 26f extending fowardly of the hollow block 26a. The angled body portion comprises two wings 26g that each have an inwardly angled hole 26h therethrough to define a divergent path. The inwardly angled holes 26g are used to drill respective holes in the patients tibia 18 into which a pin is inserted into each hole to retain the distal end 22g of the positioning jig 22 in the desired position.

The lateral adjustment portion 28 of the tool holder 24 comprises an insertion portion 28a for insertion into the opening 26b of the hollow block 26a of the longitudinal adjustment portion 26 and a tool mount attachment portion 28b. The insertion portion 28a comprises two box sections (not shown) spaced apart and sized to allow insertion into the two chambers defined by the hollow block 26a of the longitudinal adjustment portion 26. One of said box sections is provided with a position indicator (not shown) for use in conjunction with the position guide of the longitudinal adjustment portion 26. The other of said box sections is provided with a means of engagement with the mechanical fastener 26e so that the longitudinal position of the tool holder 24 can be set.

The tool mount attachment portion 28b of the lateral adjustment portion 28 of the tool holder 24 comprises a hollow box section having an obround aperture 28c in its top surface 28d and a channel 28e in its front surface 28f. A position guide (not shown) is positioned adjacent the obround aperture 28c. The obround aperture is configured to engage with a mechanical fastener 28g forming part of the tool mount 30. The mechanical fastener 28g acts as a position indicator in combination with the position guide. The channel 28e extends laterally across the front surface of the tool mount attachment portion 28b of the lateral adjustment portion 28 of the tool holder 24 and comprises a substantially symmetrical mounting guide 28h extending forwardly either side of the channel 28e opening.

The tool mount 30 of the tool holder comprises a mounting formation 30a in the form of two channels 30b, 30c defined by a T-shaped protrusion 30d and symmetric edge guards 30e. The mounting formation 30a is spaced apart from the lateral adjustment portion 28 of the tool holder 24 by an angled body 30f. The tool mount 30 is configured to be affixed on to the lateral adjustment portion 28 of the tool mount 24 by way of two lateral channels 30g substantially conforming to the profile of the symmetrical mounting guide 28h of the lateral adjustment portion of the tool holder 24.

The mounting formation 30a of the tool mount 30 of the tool holder is configured to receive a plurality of different tools. Once a portion of a tool has been received by the two channels 30b, 30c defined in the tool mount, two mechanical fasteners 30h, 30j, i.e. hex head bolts, are tightened to compress a portion of the mounting formation against the tool thus preventing movement of the tool along the channels 30b, 30c.

The final position of the tool positioning jig 22 is verified by imaging, i.e. x-ray, before or after the surgeon makes an incision into flesh surrounding the person's ankle to expose the tibia 18 and talus 20.

The first tool to be mounted onto the tool positioning jig 22 is a cyclical saw (not shown). The cyclical saw is used to create a cut, called a distal tibial cut, through the person's tibia 18. The cyclical saw is used to cut a 5° cut through the person's tibia 18. The cyclical saw is then removed from the positioning jig 22 by releasing the mechanical fasteners 30h, 30j and sliding the cyclical saw up the channels 30b, 30c and clear of the tool holder 24.

In order for the artificial ankle joint 10 to be implanted, a second cut, known as a superior talar cut, is required, this time through the person's talus 20, to create a gap of precisely thirteen millimetres between the person's tibia 18 and the person's talus 20 in order to accommodate the artificial ankle joint 10. It is critical that the thirteen millimetre gap is cut to a tightly controlled tolerance.

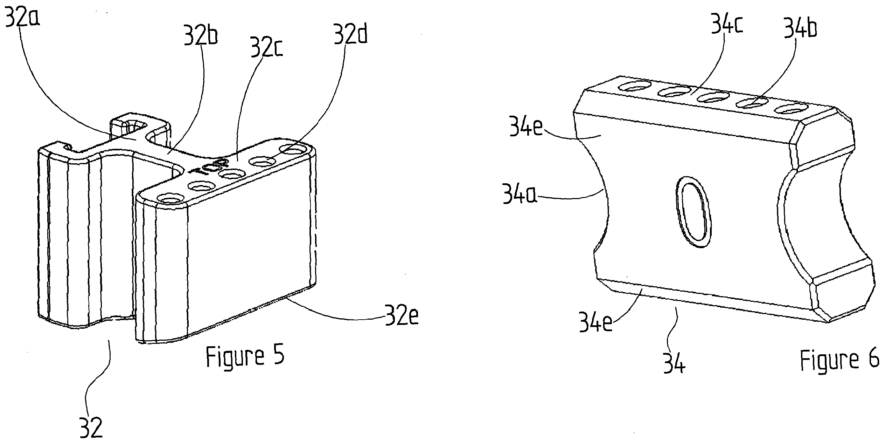

A Talus positioning guide 32, as shown in figure 5, is mounted onto the tool positioning jig 22, as has been described. The talus positioning guide 32 comprises a C-shaped mounting portion 32a for mounting onto tool holder 24 and a T-shaped extension portion 32b extending forwardly from the mounting portion 32a. The horizontal part 32c of the extension portion 32b is provided with five evenly spaced holes 32d therethrough passing from the top surface 32e to the bottom surface 32f. Although the illustrated embodiment shows five holes 32d it will be appreciated that any suitable number of holes could be used.

Holes are drilled into the person's talus 20 using the holes 32d through the talus positioning guide 32 as a guide. Although the talus positioning guide has five holes therethrough, in use only two holes are likely to be drilled into the person's talus 20. The provision of multiple holes 32d means that the talus positioning guide 32 is suitable for use on patients of different size and age. Once holes have been drilled into the person's talus 20, pins (not shown) are inserted through the holes 32d through the talus positioning guide 32 and into the holes drilled into the person's talus 20. The talus positioning guide 32 is then removed from the tool positioning jig 22, as has been described, and slid upwardly leaving the pins in place.

A talus re-section guide 34, as shown in figure 6, is then positioned over the pins and mounted on to the tool positioning jig 22, as has been described. The talus re-section guide 34 comprises a substantially rectangular block 34a of material with five holes 34b passing therethrough from the top surface 34c to the bottom surface 34d. The holes 34b substantially correspond to the position and dimension of the holes 32d in the talus positioning guidek 32. The face 34e of the talus re-section guide 34 oriented towards the person's tibia 18 is used to position a saw against for making the superior talar cut. The superior talar cut is made parallel to the distal tibial cut. At this point the tool positioning jig 22 is no longer required and can be removed from the patient by removing the talus re-section guide 34 from the tool positioning jig 22, as has been described, removing the pins inserted through the holes 26g in the wings 26h of the longitudinal adjustment portion 26 of the tool holder 24 and into the person's tibia and releasing the proximal end 22a of the tool positioning jig 22 from around the patients knee.

As described above, the tibia attachment portion 14 of the replacement ankle joint 10 comprises two tibia engagement members 14d, or kiels. Respective slots (not shown) are cut into the person's tibia 18 to accommodate said engagement members 14d.

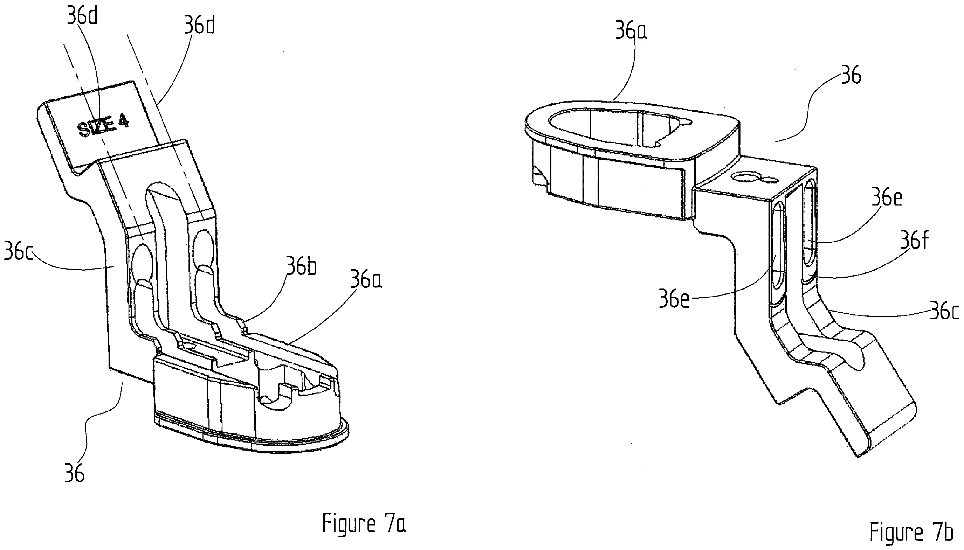

An ankle positioning jig 36, as shown in figures 7a and 7b, is used to ensure slots are cut accurately and in the correct position in the person's tibia 18b. The ankle positioning jig 36 comprises a wedge portion 36a configured to fit in the gap created between the person's tibia 18 and talus 20 by the superior talar and distal tibial cuts. The wedge portion 36a is inserted into the gap to a distance limited by a backstop 36b positioned adjacent the wedge portion 36a. The ankle positioning jig further comprises a routing portion 36c and a guide portion 36g defining two drill guides 36d, as will be described.

The routing portion 36c comprises two substantially oval apertures 36e therethrough and a cutting face 36f against which a routing tool (not shown) can rest. The routing portion 36c extends substantially perpendicularly from the wedge portion 36a of the tibia positioning block 36. In use, only one of the oval holes 36e through the tibia positioning block 36 is used to cut a slot through the tibia 18 at any one time. The depth of the slot is limited to how far the routing tool can penetrate into the person's tibia bone 18. The routing tool is adjustable to cut several pre-determined different depths. The cutting face 12f thus ensures that a slot of an accurate depth is cut into the person's tibia bone 18.

Once the slot has been cut, a chisel is inserted into the slot to dislodge any rough bone material. Distance markers on the body of the chisel assist a surgeon in inserting the chisel into the slots cut into the person's tibia bone 18 to a known depth.

A key (not shown), substantially corresponding to the size and shape of the slot, is inserted into the first slot through the oval aperture 36e in the tibia positioning block 36 to prevent rotational movement of the tibia positioning block 36 while the second slot is cut in the person's tibia 18. When inserted, the key also removes any loose bone material left after the slot has been routed and chiselled.

As can be seen in figure 7a, the drill guides 36d defined by the guide portion 36g pass through the routing portion 36c of the tibia routing guide 36 at an angle adjacent and above the oval apertures 36e. The drill guides are used to drill holes into the person's talus 20 after the slots have been cut into the person's tibia 18. Once the slots have been cut into the person's tibia 18, the drill will miss the tibia 18 and pass through the slots. The purpose of the holes drilled into the person's talus 20 will be described below. Once the holes have been drilled into the person's talus 20 the ankle positioning jig 36 is removed.

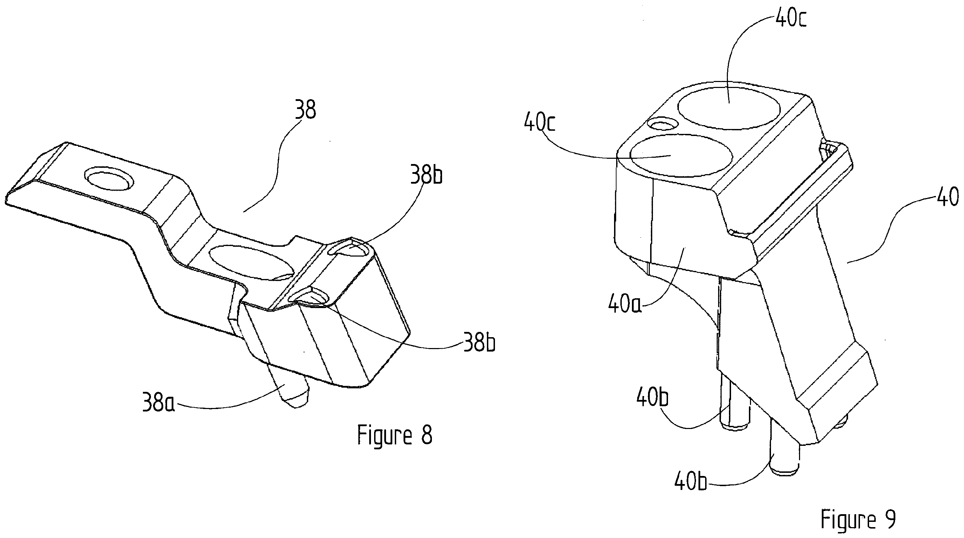

A talus drill template 38, as shown in figure 8, comprising two dowels 38a and two drill guides 38b located parallel to the dowels 38a is then positioned adjacent to the person's talus 20. The dowels 38b insert into the holes drilled into the talus, as described above in relation to the ankle positioning jig 36. Two further holes are then drilled into the person's talus 20 through the drill guides 38b to create a pattern of four holes in a substantially square, rectangular or diamond configuration in the person's talus 20.

A talus roof top preparation guide 40, as shown in figure 9, comprises a body 40a having four dowels, or pins, 40b which insert into the respective holes drilled in the person's talus 20, as described above in relation to use of ankle positioning jig 36 and talus drill template 38. The talus roof top preparation guide 40 comprises two further large diameter holes 40b used to drill two angled holes into the person's talus 20. The talus roof top preparation guide 40 further comprises an inclined face 40c against which a cyclical saw (not shown) can be positioned to re-section the person's talus 20.

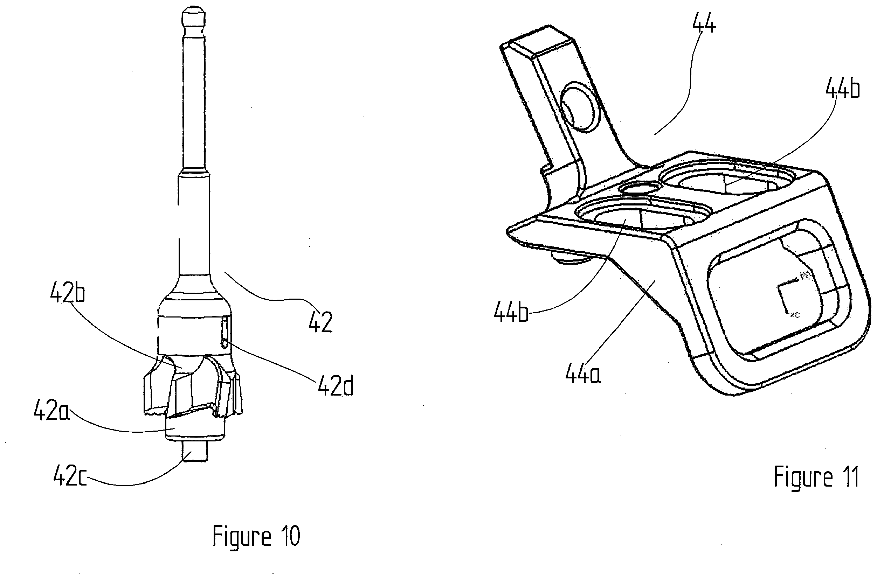

A two-stage cutting tool 42, as shown in figure 10, is used to remove further bone material surrounding the two large diameter holes 40c cut into the person's talus 20, as described above in relation to use of the talus roof top preparation guide 40, in order to create an angled portion of the person's talus 20 which forms an apex with the re-section made by the cyclical saw, as described above.

The two-stage cutting tool 42 comprises a shaft 42a having a diameter substantially similar to the greater diameter holes pre-drilled in the person's talus 20 and a router, or similar cutting tool, 42b positioned around the shaft 42a. The shaft 42a is also provided with a drill bit 42c extending axially therefrom and having a diameter smaller than the diameter of the shaft 42a. The drill bit 42c is used to drill respective holes at the base of each of the large diameter holes cut in the person's talus 20 through the roof top preparation guide 40.

The router 42b is movable between a first configuration in which the router 42b is not in contact with bone material and the shaft 42a extends from the router 42b and a second configuration in which the router 42b makes contact with bone material and the router 42b substantially surrounds the entire shaft 42a. The router 42b is biased in the first configuration by a sprung loaded mechanism 42d. An example of such a mechanism comprises a spring (not shown) positioned axially between the shaft 42a and the router 42b. When a user pushes down on the cutting tool 42, the spring force would be overcome to move the router 42b into the second configuration. When the user releases the cutting tool 42, the spring would urge the router 42b back into the first configuration. In the first configuration the shaft 42a extends beyond the router 42b and in the second configuration the shaft 42a is substantially surrounded by the router 42b.

In use, the shaft 42a is inserted into one of the large diameter holes cut in the person's talus 20. The router 42b is then depressed to move it into the second configuration such that bone material can be removed by the router 42b. After use, the router 42b reverts to the first configuration when pressure by the surgeon is removed. At this stage, the talus 20 has an apex over which a talus routing guide 44 is fitted.

The talus routing guide 44, as shown in figure 11, is fixed in place by at least two pins passing therethrough and into respective holes pre-drilled into the talus by the drill bit 42c of the two-stage cutting tool 42. The talus routing guide 44 comprises a body 44a having first and second angled faces conforming substantially to the shape of the apex of the person's talus 20 and two substantially oval apertures 44b therethrough which overlay the person's talus 20. The outer surface of at least one of the first and second angled faces acts as a cutting surface for a router to rest against.

The router is used to cut a slot into the person's talus 20 through one of the substantially oval apertures 44b. The depth of the slot is limited to how far the routing tool can penetrate into the person's tibia bone 18. The routing tool is adjustable to cut several pre-determined different depths. The cutting face 12f thus ensures that a slot of an accurate depth is cut into the person's tibia bone 18.

A chisel is inserted into the slot cut into the person's talus 20 to dislodge any rough bone material. Distance markers on the body of the chisel assist a surgeon in inserting the chisel into the slots cut into the person's tibia bone 18 to a known depth.

A key is then inserted into the slot to prevent rotational movement of the talus routing guide 44 and remove any loose bone material from the slot. The other substantially oval aperture 44b is then used to cut the second slot into the person's talus 20. The talus routing guide 44 can then be removed.

A manipulator 46, as shown in figure 12 is used to position the artificial ankle joint 10 within the gap created between the person's tibia and talus by the superior talar and distal tibia cuts, as described above. The manipulator 46 comprises a handle portion 46a, an artificial ankle joint holder 46b, two diametrically opposed mounting portions 46c and, optionally a third mounting portion 46d angularly spaced apart from the diametrically opposed mounting portions 46c by approximately ninety degrees. The diametrically opposed mounting portions 46c and the third mounting portion, if present, of the manipulator 46 engage with the mounting portions 12h of the talus attachment portion 14 of the artificial ankle joint 10.

Each of the mounting portions 46b, 46c of the manipulator 46 are sprung loaded or resiliently deformable such that as the artificial ankle joint 10 is inserted into the manipulator 46, the mounting portions 46b, 46c thereof deform to allow access to the artificial ankle joint 10 before reverting to an unstressed state to engage with the talus attachment portion 14 of the artificial ankle joint 10.

The configuration of the artificial ankle joint 10 means that the manipulator 46 can only pick the artificial ankle 10 joint up in one orientation. The manipulator 46 has a curvilinear profile substantially corresponding to the profile of an artificial ankle joint 10. The profile of the manipulator 46 is non-symmetrical meaning that the artificial ankle joint 10 can only be inserted into the manipulator 46 one way round.

The engagement members on the tibia attachment portion are aligned with the slots in the tibia 18 and the engagement members on the talus attachment portion are aligned with the slots in the talus 20. Each portion of the artificial ankle joint 10 is then impacted by a percussion tool, or pressed, to urge the engagement members into respective slots in the person's tibia 18 and talus 20. The engagement members are held in position by an interference fit between the engagement members and their respective slots.

## Claims

1. Apparatus for replacing a human ankle joint, the apparatus comprising:
a tool holder (24); a tool positioning jig (22) for setting a position of the tool holder (24) relative to a part of a person's anatomy;
a cyclical saw removably mountable to the tool holder for making a distal tibial cut though the person's tibia bone;
a talus positioning guide (32) removably mountable to the tool holder for setting a position of location holes to be drilled in the person's talus bone; a talus re-section guide (34), alignable with the location holes to be drilled in the person's talus bone, for providing a cutting face for making a superior talar cut through the person's talus bone;
an ankle alignment jig (36) for setting the position of one or more slots in the person's tibia bone, the said one or more slots being configured to receive a first portion of an artificial ankle joint, and for setting the position of one or more guide holes to be drilled in the person's talus bone in order to facilitate medial, lateral, posterior and anterior alignment of the first portion of the artificial ankle joint with a second portion of the artificial ankle joint (10);
a talus drill template (38) comprising one or more dowels (38a), or pins, configured to be received by the one or more guide holes to be drilled in the person's talus bone, the talus drill template further comprising one or more drill guides (38b) for setting the position of at least one further hole to be drilled in the person's talus bone;
a talus rooftop preparation guide (40) comprising at least two dowels (40b), or pins, configured to be received respectively by the one or more guide holes to be drilled in the person's talus bone and by the at least one further hole to be drilled in the person's talus bone, the rooftop preparation guide further comprising two large diameter holes (40c) for receiving a cutting tool (42) for removing a portion of the person's talus bone;
a talus routing guide (44) for setting the position of one or more slots in the person's talus bone, said one or more slots being configured to receive the second portion of the artificial ankle joint and
a manipulator (46) for positioning the artificial ankle joint between the person's tibia and talus bones, the manipulator comprising a handle portion (46a) and a body portion (46b),
**characterised in that** the body portion of the manipulator comprises at least one mounting point (46c) configured to engage with the artificial ankle joint and a direction guide configured to prevent the artificial ankle joint from being incorrectly inserted therein.

2. Apparatus for replacing a human ankle joint according to claim 1, wherein the talus positioning guide (32) comprises an extension portion (32b) and a location portion (32a), wherein the extension portion positions the location portion a pre-determined distance away from the tool holder (24) and the location portion comprises a plurality of holes (32d) therethrough for setting the position of the location holes in the person's talus bone.

3. Apparatus for replacing a human ankle joint according to claim 1 or claim 2, wherein the talus roof top preparation guide (40) comprises a body portion (40a) containing the two large diameter holes (40b) therethrough for receiving the cutting tool for removing a portion of the person's talus bone and a cutting surface at an angle relative to the body portion for creating a re-section through the person's talus bone, the removal of the portion of the person's talus bone and the re-section of the person's talus bone between them creating an apex.

4. Apparatus for replacing a human ankle joint according to claim 2, wherein the plurality of holes (40b) through the location portion of the talus positioning guide are substantially evenly spaced.

5. Apparatus for replacing a human ankle joint according to any of claims 2 to 4, wherein the extension portion (32b) and location portion (32a) of the talus positioning block define a T-shape therebetween, wherein the extension portion defines the vertical geometry of the T-shape and the location portion defines the horizontal geometry of the T-shape.

6. Apparatus for replacing a human ankle joint according to any of claims 1 to 5, wherein the talus re-section guide (34) comprises a substantially rectangular block (34a) having a plurality of holes (34b) therethrough, said holes being spaced apart substantially evenly and substantially corresponding in position and size to the plurality of holes through the location portion of the talus positioning guide (32).

7. Apparatus for replacing a human ankle joint according to any preceding claim, wherein the ankle positioning jig (36) comprises a wedge portion (36a) for insertion between the person's tibia and talus bones;
a routing portion (36c) for sitting adjacent the person's tibia, said routing portion comprising the at least one aperture for setting the position of the one or more slots in the person's tibia; and
a guide portion (36g) containing the one or more guide holes for medially and laterally aligning the first portion of the artificial ankle joint (10) with the second portion of the artificial ankle joint

8. Apparatus for replacing a human ankle joint according to claim 7, wherein the wedge portion (36a) of the ankle positioning jig (36) comprises a backstop (36b) to limit the depth of insertion of the ankle positioning jig the person's tibia and talus bones.

9. Apparatus for replacing a human ankle joint according to any preceding claim, wherein the at least one aperture through the routing portion (36c) of the ankle positioning jig (36) comprises two oval apertures.

10. Apparatus for replacing a human ankle joint according to any of claims 7 to 9, wherein the routing portion (36c) of the ankle positioning jog (36) extends substantially perpendicularly from the wedge portion (36a) of said ankle positioning jig.

11. Apparatus for replacing a human ankle joint according to any of claims 7 to 10, wherein the guide portion (36g) of the ankle positioning jig (36) is angled relative to the routing portion (36c) of the ankle positioning jig such that the centre line of the guide holes passes through a portion of the at least one aperture of the routing portion of the ankle positioning jig.

12. Apparatus for replacing a human ankle joint according to any preceding claim, wherein the talus drill template (38) comprises two dowels (38a), or pins, extending angularly therefrom, and two drill guides (38b) arranged such that the dowels, or pins, and the drill guides define between them a square, rectangular or diamond configuration.

13. Apparatus for replacing a human ankle joint according to any preceding claim, wherein the talus routing guide (44) comprises a first angled face and a second angled face defining an apex between them configured to substantially correspond to a lateral cross-section of the person's talus bone.

14. A tool (46) configured for positioning an artificial ankle joint between a person's tibia and talus bones, the tool comprising:
a handle portion (46a) and a body portion (46b), **characterised in that** the body portion further comprises a pair of diametrically opposed mounting points (46c) configured to engage with an artificial ankle joint, and a direction guide configured to prevent the artificial ankle joint from being incorrectly inserted therein.

15. A tool (46) according to claim 14, wherein the direction guide comprises a further mounting point (46d) on the body portion (46) of the manipulator.

## Patentansprüche

1. Apparat zum Ersetzen eines menschlichen Knöchelgelenks, wobei der Apparat Folgendes beinhaltet:
einen Werkzeughalter (24);
eine Werkzeugpositionierungsvorrichtung (22) zum Einstellen einer Position des Werkzeughalters (24) relativ zu einem Teil der Anatomie einer Person;
eine zyklische Säge, die am Werkzeughalter zum Herstellen eines distalen tibialen Schnitts durch den Tibiaknochen des Patienten entfernbar montierbar ist;
eine Taluspositionierungsführung (32), die am Werkzeughalter entfernbar montierbar ist, zum Einstellen einer Position von in den Talusknochen des Patienten zu bohrenden Fixierungslöchern;
eine Talusresektionsführung (34), die auf die in den Talusknochen des Patienten zu bohrenden Fixierungslöcher ausrichtbar ist, zum Bereitstellen einer Schnittfläche zum Herstellen eines superioren, talaren Schnitts durch den Talusknochen des Patienten;
eine Knöchelausrichtungsvorrichtung (36) zum Einstellen der Position von einem oder mehreren Schlitzen im Tibiaknochen des Patienten, wobei der eine oder die mehreren Schlitze konfiguriert sind, einen ersten Abschnitt eines künstlichen Knöchelgelenks aufzunehmen, und zum Einstellen der Position von einem oder mehreren in den Talusknochen der Person zu bohrenden Führungslöchern, um mediale, laterale, posteriore und anteriore Ausrichtung des ersten Abschnitts des künstlichen Knöchelgelenks auf einen zweiten Abschnitt des künstlichen Knöchelgelenks (10) zu erleichtern;
eine Talusbohrschablone (38), die einen oder mehrere Dübel (38a) oder Stifte beinhaltet, die konfiguriert sind, von dem einen oder den mehreren in den Talusknochen der Person zu bohrenden Führungslöchern aufgenommen zu werden, wobei die Talusbohrschablone ferner eine oder mehrere Bohrführungen (38b) zum Einstellen der Position von mindestens einem weiteren in den Talusknochen der Person zu bohrenden Loch beinhaltet;
eine Talusdachvorbereitungsführung (40), die mindestens zwei Dübel (40b) oder Stifte beinhaltet, die konfiguriert sind, von dem einen oder den mehreren in den Talusknochen der Person zu bohrenden Führungslöchern und von dem mindestens einen weiteren in den Talusknochen der Person zu bohrenden Loch aufgenommen zu werden, wobei die Dachvorbereitungsführung ferner zwei Löcher mit großem Durchmesser (40c) zum Aufnehmen eines Schneidwerkzeugs (42) zum Entfernen eines Abschnitts des Talusknochen der Person beinhaltet;
eine Talusroutingführung (44) zum Einstellen der Position von einem oder mehreren Schlitzen im Talusknochen der Person, wobei der eine oder die mehreren Schlitze konfiguriert sind, den zweiten Abschnitt des künstlichen Knöchelgelenks aufzunehmen, und
einen Manipulator (46) zum Positionieren des künstlichen Sprunggelenks zwischen dem Tibia-und Talusknochen der Person, wobei der Manipulator einen Griffabschnitt (46a) und einen Körperabschnitt (46b) beinhaltet,
**dadurch gekennzeichnet, dass** der Körperabschnitt des Manipulators mindestens einen Anbringungspunkt (46c), der konfiguriert ist, das künstliche Sprunggelenk in Eingriff zu nehmen, und eine Richtungsführung, die konfiguriert ist, zu verhindern, dass das künstliche Sprunggelenk inkorrekt darin eingeführt wird, beinhaltet.

2. Apparat zum Ersetzen eines menschlichen Knöchelgelenks gemäß Anspruch 1, wobei die Taluspositionierungsführung (32) einen Verlängerungsabschnitt (32b) und einen Fixierungsabschnitt (32a) beinhaltet, wobei der Verlängerungsabschnitt den Fixierungsabschnitt einen vorbestimmten Abstand vom Werkzeughalter (24) entfernt positioniert und der Fixierungsabschnitt eine Vielzahl von Löchern (32d) durch diesen zum Einstellen der Position der Fixierungslöcher im Talusknochen der Person beinhaltet.

3. Apparat zum Ersetzen eines menschlichen Knöchelgelenks gemäß Anspruch 1 oder Anspruch 2, wobei die Talusdachvorbereitungsführung (40) einen Körperabschnitt (40a) beinhaltet, der die zwei Löcher mit großem Durchmesser (40b) durch diesen zum Aufnehmen des Schneidwerkzeugs zum Entfernen eines Abschnitts des Talusknochen der Person und eine Schneidfläche mit einem Winkel relativ zum Körperabschnitt enthält, zum Erzeugen einer Resektion durch den Talusknochen der Person, wobei Entfernung des Talusknochen der Person und die Resektion des Talusknochen der Person dazwischen eine Spitze erzeugt.

4. Apparat zum Ersetzen eines menschlichen Knöchelgelenks gemäß Anspruch 2, wobei die Vielzahl von Löchern (40b) durch den Fixierungsabschnitt der Taluspositionierungsführung im Wesentlichen gleichmäßig beabstandet sind.

5. Apparat zum Ersetzen eines menschlichen Knöchelgelenks gemäß einem der Ansprüche 2 bis 4, wobei der Verlängerungsabschnitt (32b) und der Fixierungsabschnitt (32a) des Taluspositionierungsblocks eine T-Form dazwischen definieren, wobei der Verlängerungsabschnitt die vertikale Geometrie der T-Form definiert und der Fixierungsabschnitt die horizontale Geometrie der T-Form definiert.

6. Apparat zum Ersetzen eines menschlichen Knöchelgelenks gemäß einem der Ansprüche 1 bis 5, wobei die Talusresektionsführung (34) einen im Wesentlichen rechtwinkligen Block (34a) beinhaltet, der eine Vielzahl von Löchern (34b) durch diesen aufweist, wobei die Löcher im Wesentlichen gleichmäßig voneinander beabstandet sind und im Wesentlichen in Position und Größe der Vielzahl von Löchern durch den Fixierungsabschnitt der Taluspositionierungsführung (32) entsprechen.

7. Apparat zum Ersetzen eines menschlichen Knöchelgelenks gemäß einem vorhergehenden Anspruch, wobei die Knöchelpositionierungsvorrichtung (36) Folgendes beinhaltet:
einen Keilabschnitt (36a) zum Einführen zwischen dem Tibia- und Talusknochen der Person;
einen Routingabschnitt (36c) zum Sitzen neben der Tibia der Person, wobei der Routingabschnitt die mindestens eine Öffnung zum Einstellen der Position des einen oder der mehreren Schlitze der Tibia der Person beinhaltet; und
einen Führungsabschnitt (36g), der das eine oder die mehreren Führungslöcher zum medialen und lateralen Ausrichten des ersten Abschnitts des künstlichen Knöchelgelenks (10) auf den zweiten Abschnitt des künstlichen Knöchelgelenks beinhaltet.

8. Apparat zum Ersetzen eines menschlichen Knöchelgelenks gemäß Anspruch 7, wobei der Keilabschnitt (36a) der Knöchelpositionierungsvorrichtung (36) einen Anschlag (36b) beinhaltet, um die Tiefe der Einführung der Knöchelpositionierungsvorrichtung der Tibia- und Talusknochen der Person zu begrenzen.

9. Apparat zum Ersetzen eines menschlichen Knöchelgelenks gemäß einem vorhergehenden Anspruch, wobei die mindestens eine Öffnung durch den Routingabschnitt (36c) der Knöchelpositionierungsvorrichtung (36) zwei ovale Öffnungen beinhaltet.

10. Apparat zum Ersetzen eines menschlichen Knöchelgelenks gemäß einem der Ansprüche 7 bis 9, wobei sich der Routingabschnitt (36c) der Knöchelpositionierungsvorrichtung (36) im Wesentlichen senkrecht vom Keilabschnitt (36a) der Knöchelpositionierungsvorrichtung erstreckt.

11. Apparat zum Ersetzen eines menschlichen Knöchelgelenks gemäß einem der Ansprüche 7 bis 10, wobei der Führungsabschnitt (36g) der Knöchelpositionierungsvorrichtung (36) relativ zum Routingabschnitt (36c) der Knöchelpositionierungsvorrichtung gewinkelt ist, sodass die Mittellinie der Führungslöcher durch einen Abschnitt der mindestens einen Öffnung des Routingabschnitts der Knöchelpositionierungsvorrichtung verläuft.

12. Apparat zum Ersetzen eines menschlichen Knöchelgelenks gemäß einem vorhergehenden Anspruch, wobei die Talusbohrschablone (38) zwei Dübel (38a) oder Stifte, die sich winkelmäßig davon erstrecken, und zwei Bohrführungen (38b), die so angeordnet sind, dass die Dübel oder Stifte, und die Bohrführungen dazwischen eine quadratische, rechtwinklige oder rautenförmige Konfiguration definieren, beinhaltet.

13. Apparat zum Ersetzen eines menschlichen Knöchelgelenks gemäß einem vorhergehenden Anspruch, wobei die Talusroutingführung (44) eine erste gewinkelte Fläche und eine zweite gewinkelte Fläche beinhaltet, die dazwischen eine Spitze definieren, die im Wesentlichen konfiguriert ist, um einem lateralen Querschnitt des Talusknochen der Person zu entsprechen.

14. Werkzeug (46), das zum Positionieren eines künstlichen Sprunggelenks zwischen dem Tibia- und Talusknochen einer Person konfiguriert ist, wobei das Werkzeug Folgendes beinhaltet:
einen Griffabschnitt (46a) und einen Körperabschnitt (46b), **dadurch gekennzeichnet, dass** der Körperabschnitt ferner ein Paar diametrisch entgegengesetzte Anbringungspunkte (46c), die konfiguriert sind, ein künstliches Sprunggelenk in Eingriff zu nehmen, und eine Richtungsführung, die konfiguriert ist, zu verhindern, dass das künstliche Sprunggelenk inkorrekt darin eingeführt wird, beinhaltet.

15. Werkzeug (46) gemäß Anspruch 14, wobei die Richtungsführung einen weiteren Anbringungspunkt (46d) am Körperabschnitt (46) des Manipulators beinhaltet.

## Revendications

1. Appareil pour remplacer une articulation de cheville humaine, l'appareil comprenant :
un porte-outil (24) ; un gabarit de positionnement d'outil (22) pour fixer une position du porte-outil (24) par rapport à une partie de l'anatomie d'une personne ;
une scie à mouvement cyclique pouvant être montée de manière amovible sur le porte-outil pour effectuer une coupe tibiale distale dans le tibia de la personne ;
un guide de positionnement de l'astragale (32) pouvant être monté de manière amovible sur le porte-outil pour fixer une position de trous de positionnement devant être percés dans l'astragale de la personne ;
un guide de résection de l'astragale (34), pouvant s'aligner avec les trous de positionnement devant être percés dans l'astragale de la personne, afin de fournir une face de coupe pour effectuer une coupe dans la partie supérieure de l'astragale de la personne ;
un gabarit d'alignement de la cheville (36) pour fixer la position d'une ou plusieurs fentes dans le tibia de la personne, la ou lesdites fentes étant configurées pour recevoir une première partie d'une articulation de cheville artificielle, et pour fixer la position d'un ou plusieurs trous de guidage devant être percés dans l'astragale de la personne afin de faciliter l'alignement médial, latéral, postérieur et antérieur de la première partie de l'articulation de cheville artificielle avec une deuxième partie de l'articulation de cheville artificielle (10) ;
un gabarit de perçage de l'astragale (38) comprenant une ou plusieurs goupilles (38a), ou broches, configurées pour être reçues par le ou les trous de guidage devant être percés dans l'astragale de la personne, le gabarit de perçage de l'astragale comprenant en outre un ou plusieurs guides de perçage (38b) pour fixer la position d'au moins un autre trou devant être percé dans l'astragale de la personne ;
un guide de préparation de toit d'astragale (40) comprenant au moins deux goupilles (40b), ou broches, configurées pour être reçues respectivement par un ou plusieurs trous de guidage devant être percés dans l'astragale de la personne et par le ou les autres trous devant être percés dans l'astragale de la personne, le guide de préparation de toit comprenant en outre deux trous de grand diamètre (40c) pour recevoir un outil de coupe (42) pour enlever une partie de l'astragale de la personne ;
un guide d'acheminement de l'astragale (44) pour fixer la position d'une ou plusieurs fentes dans l'astragale de la personne, ladite ou lesdites fentes étant configurées pour recevoir la deuxième partie de l'articulation de cheville artificielle, et
un manipulateur (46) pour positionner l'articulation de cheville artificielle entre le tibia et l'astragale de la personne, le manipulateur comprenant une partie formant une poignée (46a) et une partie formant un corps (46b),
**caractérisé en ce que** la partie formant le corps du manipulateur comprend au moins un point de montage (46c) configuré pour s'engager avec l'articulation de cheville artificielle et un guide de direction configuré pour empêcher l'articulation de cheville artificielle d'y être incorrectement insérée.

2. Appareil pour remplacer une articulation de cheville humaine selon la revendication 1, dans lequel le guide de positionnement de l'astragale (32) comprend une partie de prolongement (32b) et une partie de positionnement (32a), la partie de prolongement positionnant la partie de positionnement à une distance prédéterminée du porte-outil (24) et la partie de positionnement comprenant une pluralité de trous (32d) qui la traversent pour fixer la position des trous de positionnement dans l'astragale de la personne.

3. Appareil pour remplacer une articulation de cheville humaine selon la revendication 1 ou la revendication 2, dans lequel le guide de préparation de toit de l'astragale (40) comprend une partie formant un corps (40a) contenant les deux trous de grand diamètre (40b) qui la traversent pour recevoir l'outil de coupe pour enlever une partie de l'astragale de la personne et une surface de coupe en biais par rapport à la partie formant corps pour créer une résection à travers l'astragale de la personne, l'enlèvement de la partie de l'astragale de la personne et la résection de l'astragale de la personne formant entre eux un apex.

4. Appareil pour remplacer une articulation de cheville humaine selon la revendication 2, dans lequel la pluralité de trous (40b) traversant la partie de positionnement du guide de positionnement de l'astragale sont essentiellement uniformément espacés.

5. Appareil pour remplacer une articulation de cheville humaine selon l'une quelconque des revendications 2 à 4, dans lequel la partie de prolongement (32b) et la partie de positionnement (32a) du bloc de positionnement de l'astragale définissent entre elles une forme de T, dans laquelle la partie de prolongement définit la barre verticale du T et la partie de positionnement définit la barre horizontale du T.

6. Appareil pour remplacer une articulation de cheville humaine selon l'une quelconque des revendications 1 à 5, dans lequel le guide de résection de l'astragale (34) comprend un bloc essentiellement rectangulaire (34a) ayant une pluralité de trous (34b) qui le traversent, lesdits trous étant espacés essentiellement uniformément et correspondant essentiellement par la position et la taille à la pluralité de trous traversant la partie de positionnement du guide de positionnement de l'astragale (32).

7. Appareil pour remplacer une articulation de cheville humaine selon l'une quelconque des revendications précédentes, dans lequel le gabarit de positionnement de cheville (36) comprend une partie en coin (36a) devant être insérée entre le tibia et l'astragale de la personne ;
une partie d'acheminement (36c) devant se situer à proximité du tibia de la personne, ladite partie d'acheminement comprenant la ou les ouvertures pour fixer la position de la ou des fentes dans le tibia de la personne ; and
une partie de guidage (36g) contenant le ou les trous de guidage pour aligner médialement et latéralement la première partie de l'articulation de cheville artificielle (10) avec la deuxième partie de l'articulation de cheville artificielle.

8. Appareil pour remplacer une articulation de cheville humaine selon la revendication 7, dans lequel la partie en coin (36a) du gabarit de positionnement de la cheville (36) comprend une butée (36b) pour limiter la profondeur d'insertion du gabarit de positionnement de la cheville dans le tibia et l'astragale de la personne.

9. Appareil pour remplacer une articulation de cheville humaine selon l'une quelconque des revendications précédentes, dans lequel au moins une ouverture dans la partie d'acheminement (36c) du gabarit de positionnement de la cheville (36) comprend deux ouvertures ovales.

10. Appareil pour remplacer une articulation de cheville humaine selon l'une quelconque des revendications 7 à 9, dans lequel la partie d'acheminement (36c) du gabarit de positionnement de la cheville (36) s'étend essentiellement perpendiculairement à partir de la partie en coin (36a) dudit gabarit de positionnement de la cheville.

11. Appareil pour remplacer une articulation de cheville humaine selon l'une quelconque des revendications 7 à 10, dans lequel la partie de guidage (36g) du gabarit de positionnement de la cheville (36) est en biais par rapport à la partie d'acheminement (36c) du gabarit de positionnement de la cheville de telle sorte que l'axe des trous de guidage passe par une partie de la ou des ouvertures de la partie d'acheminement du gabarit de positionnement de la cheville.

12. Appareil pour remplacer une articulation de cheville humaine selon l'une quelconque des revendications précédentes, dans lequel le gabarit de perçage de l'astragale (38) comprend deux goupilles (38a), ou broches, s'étendant en biais à partir du gabarit, et deux guides de perçage (38b) agencés de telle sorte que les goupilles, ou broches, et les guides de perçage définissent entre eux une configuration carrée, rectangulaire ou en losange.

13. Appareil pour remplacer une articulation de cheville humaine selon l'une quelconque des revendications précédentes, dans lequel le guide d'acheminement de l'astragale (44) comprend une première face en biais et une deuxième face en biais définissant entre elles un apex configuré pour correspondre essentiellement à une section transversale de l'astragale de la personne.

14. Outil (46) configuré pour positionner une articulation de cheville artificielle entre le tibia et l'astragale d'une personne, l'outil comprenant :
une partie formant une poignée (46a) et une partie formant un corps (46b), **caractérisées en ce que** la partie formant le corps comprend en outre une paire de points de montage diamétralement opposés (46c) configurés pour s'engager avec une articulation de cheville artificielle, et un guide de direction configuré pour empêcher l'articulation de cheville artificielle d'y être insérée incorrectement.

15. Outil (46) selon la revendication 14, dans lequel le guide de direction comprend un autre point de montage (46d) sur la partie formant le corps (46) du manipulateur.
